Europäisches Patentamt

(19) European Patent Office

Office européen des brevets

(11) Publication number: **0 327 396**
**A2**

# EUROPEAN PATENT APPLICATION

(21) Application number: **89301100.7**

(51) Int. Cl.⁴: **G 01 N 33/48**

(22) Date of filing: **03.02.89**

(30) Priority: **05.02.88 US 152470**

(43) Date of publication of application:
**09.08.89 Bulletin 89/32**

(84) Designated Contracting States: **DE ES FR GB IT**

(71) Applicant: **GUARDIAN TECHNOLOGIES, INC.**
**1009 Grant Street**
**Denver Colorado 80203 (US)**

(72) Inventor: **Comeau, Felix J. E.**
**3 Dawnridge Trail**
**Brampton Ontario (CA)**

**Kupferschmidt, Gerald J.**
**2225 Munns' Avenue**
**Oakville, Ontario (CA)**

**Read, Richard E.**
**719 East Center Street**
**Blanchester, Ohio (US)**

(74) Representative: **Allen, Oliver John Richard et al**
**Lloyd Wise, Tregear & Co. Norman House 105-109 Strand**
**London, WC2R 0AE (GB)**

(54) **Apparatus for delivering a breath sample to a solid state sensor.**

(57) An apparatus for delivering a gas sample such as a breath sample to a solid state sensor operating to sense the concentration of a constituent of the sample such as alcohol while the sensor is maintained within a predetermined temperature range is disclosed. The sensor is disposed within a sensor chamber which communicates by way of a small bleed passageway with a second chamber formed in a conduit having an inlet upstream of the second chamber and an exhaust downstream thereof. The pressure within the second chamber is passively regulated to lie within a predetermined range over an expected range of inlet pressures. This regulation is accomplished by means of flow restrictors disposed between the second chamber and its inlet and exhaust and results in a bleed flow to the sensor through the bleed passageway which is sufficient to ensure an accurate reading within a predetermined sampling interval while further ensuring that the bleed flow does not cool the sensor below its operating temperature range. The lower end of the pressure range at which the second chamber is regulated is selected to correspond to a minimum flow sufficient to provide a deep lung breath sample when that minimum flow is continued with out interruption for a period at least equal to a predetermined sampling interval. This minimum flow can be sensed for example by a pressure sensor operatively disposed in communication with the second chamber. To ensure that the pressure sensor cannot indicate the presence of the minimum flow when the exhaust is blocked, the invention further contemplates connecting the pressure sensor differentially across one of the flow restrictors separating the second chamber from its inlet and exhaust.

FIG.1

## Description

## APPARATUS FOR DELIVERING A BREATH SAMPLE TO A SOLID STATE SENSOR

The present invention relates to breath sobriety testing. More particularly, the present invention relates to an apparatus for receiving a flow of exhaled breath from an individual and delivering a sample of the breath to a solid state alcohol sensor.

It is known that the amount of alcohol present in a person's bloodstream can be determined by measuring the concentration of alcohol vapour present in the gas expired from the alveoli of the lungs. This principle has been used in a wide variety of apparatus commonly referred to as breath analyzers which are intended to accurately measure blood alcohol content (B.A.C.) based on a breath sample. An example of such a breath testing system is disclosed in U.S. Patent 3,764,270.

Breath alcohol analysis has also been applied to great advantage in the development of devices commonly referred to as "interlocks" which are intended to prevent the operation of vehicles, industrial machinery or the like by persons impaired by the effects of alcohol. Examples of interlock systems particularly as applied to vehicles, are disclosed in U.S. Patents 4,697,666 and 4,093,945 as well as U.S. Application Serial No. 06/907,881 filed September 16,1986

Breath alcohol analysis devices such as breath analyzers and interlocks are typically designed such that a test subject delivers a breath sample for analysis by blowing into a mouthpiece. From the mouthpiece, the breath flows to the vicinity of a sensing device which generates a signal correlated with the amount of alcohol vapour that is present. Because breath expired from the upper portions of the respiratory tract does not necessarily have an alcohol level proportional to that of the bloodstream, accurate results can be insured only if the sensor is exposed to a "deep lung" sample of breath when the measurement is made. This term derives from the fact that the alveoli are located at the extreme terminous of the respiratory tract. Accordingly, an extended or "deep" exhalation is required to expel breath comprised principally of the alveolar gas. As used herein and in the claims, the term "deep lung" refers to a breath sample consisting of a proportion of alveolar gas sufficient to permit a suitably accurate determination of blood alcohol content from such a sample.

If a breath alcohol analysis system is not to deliver erroneous results or be subject to evasion, deliberate of otherwise, by delivery of shallow exhalations or a series of puffes of breath expelled from upper protions of the respiratory tract, its design must require delivery of a deep lung breath sample. To this end, U.S. Patents 3,764,270 and 4,093,945 disclose means such as a pressure sensor and timer system whereby a continuous and uninterrupted flow of breath must be maintained for at least a minimum time interval. The sampling interval and the flow rate as measured by the pressure sensor or other flow sensing means are selected together to insure a deep lung sample will be given. Any interruption or insufficiency in flow during the sampling interval is sensed by the flow sensing means to prevent a reading from being given in the case of a breath analyzer or prevent starting of the vehicle in the case of a breath-analyzed ignition interlock system.

Many breath alcohol analysis devices sense the concentration of alcohol vapor in a breath sample by means of a solid state type gas sensor. Some examples of solid state gas sensors include semiconductor sensors (also known as "Taguchi" sensors in recognition of their inventor) and combustion sensors such as those of the catalytic bead type having ceramic element impregnated with a material such as platinum or palladium. As used herein, the term solid state sensor refers to a sensor having a sensing element a characteristic parameter of which (e.g. electrical resistance) changes predictably with the amount of alcohol in the vicinity of the sensor when the sensor is at a temperature lying within a certain operating range. To facilitate maintaining the sensor temperature within its operating range, solid state sensors, particularly the semiconductor variety are often equipped with a heater. The heater also permits the sensor to be "purged" by raising it to a temperature significantly above its operating range so that any alcohol or other substances previously adsorbed on its surface are oxidized and desorbed. This prepares the sensor for subsequent measurements by restoring its resistance to an equilibrium value representative of a contaminant-free state.

In some prior art breath alcohol analyzing devices utilizing solid state sensors, the sensor has been exposed directly in the breath stream being blown into the unit. This is undesirable because the breath flow can cool the sensor below its intended operating temperature range resulting in erroneous readings.

To help prevent this, U.S. Patent 4,161,875 proposes disposing the sensor within a chamber having an outlet and an inlet connected to a mouthpiece into which breath is delivered. An inlet tube connected between the mouthpiece and the chamber inlet includes a diverter valve controlled by a clock and a pressure switch. Initially, the diverter valve diverts the entire breath flow from the mouthpiece to an auxiliary outlet while an indicator light operated by the pressure switch provides means for indicating whether sufficient breath is flowing to insure a deep lung sample is delivered by the time the clock times out. When the clock times out, the diverter valve changes position to divert deep-lung flow into the sensor chamber. To avoid cooling of the sensor, the U.S. Patent 4,161,875 reveals the introduction of the breath stream into the sensor chamber along a direction selected so that the breath stream will not directly strike the sensor. This approach suffers from a number of deficiencies, however.

First, once the clock times out, the diverter valve directs the entire flow stream into the sample chamber with the sensor. Due to the relatively large mass flow of breath involved, any degree of thermal equilibrium which may have previously existed in the chamber, particularly in the vicinity of the heated sensor, is likely to be upset considerably by the influx of large amounts of breath which is at a temperature much colder than the sensor operating temperature. Accordingly, the sensor is likely to be cooled considerably despite the fact the breath stream doe not impinge directly on the sensor. This is all the more true when one considers that amount and duration of flow through the sample chamber is subject to wide variation after the diverter valve begins to direct breath toward the sensor. While the pressure switch is supposed to ensure the flow is at least a minimum value, the maximum flow rate is limited only by the lung capacity of the test subject. Similar uncontrolled variations can occur respecting the length of time over which flow through the sample chamber can continue. As the mass flow of breath through the sample chamber increases, so too does the capacity of this flow to carry off heat and cool the sensor. Thus, in addition to the inherent thermal shock of diverting the entire flow stream into the sample chamber the sensor may be cooled to an even greater degree where the subject blows hard into the mouthpiece, and/or sustains breath flow for a long period of time.

A second problem with the solution proposed by the '875 Patent is that it requires a diverter valve which can be controlled by a clock. Such a valve is undesirable since it will add cost, can malfunction or leak and requires extra space to accommodate its placement.

A third problem with the apparatus of the '875 Patent is that it permits a test subject to easily circumvent an accurate test. This can be done by simply blocking the auxiliary outlet. When the subject begins to blow into the mouthpiece, the diverter valve will initially direct flow to the auxiliary outlet. With that outlet blocked, the pressure switch will indicate adequate flow even though almost no breath is actually flowing. Therefore, the subject need not deliver a deep lung sample since the upper respiratory tract has not been adequately exhausted. When the diverter valve switches, breath that is not deep lung breath will flow into the sample chamber and an erroneous measurement will result.

In view of the foregoing problems it is an object of the present invention to provide an apparatus for delivering a breath sample to a solid state sensor in a manner which delivers a sufficient amount of flow to the sensor to permit accurate measurements while ensuring that the sensor is not cooled below its operating temperature range regardless of how forcefully or how long the test subject blows when delivering a breath sample.

Apparatus in accordance with the present invention comprises a solid states sensor disposed within a sensor chamber which communicates by way of a small bleed passageway with a second chamber formed in a conduit. The conduit has an inlet upstream of the second chamber for receiving breath flow and an outlet downstream of the second chamber serving as an exhaust. A first restricted flow passage is disposed between the inlet and the second chamber and a second restricted flow passage is disposed between the second chamber and the outlet. The flow restrictions within first and second passages are sized to create a predetermined minimum pressure within the second chamber when the flow through the chamber at least equals a corresponding minimum flow. The minimum flow is selected to be sufficient to provide a deep lung sample if at least that level of flow is maintained for at least a given sampling time interval. The restricted passages are further sized to limit the pressure in the second chamber to a maximum expected value. The size of bleed passageway is selected in accordance with the predetermined minimum pressure and the maximum expected pressure in the sample chamber such that the flow to the sensor chamber is sufficient to provide an accurate alcohol reading when the pressure is within the range defined by those two values. Within that range, the flow through the bleed passageway to the sensor chamber is at least great enough that the sensor is not "starved" for breath from the second chamber. This allows the sensor output to accurately reflect the alcohol content of the deep lung breath by the end of the sampling interval. At the same time, the flow through the bleed passage is not so large as to cool the sensor below its accurate operating range. The small size of the bleed passageway together with the regulated pressure within the second chamber also help reduce fluctuations in the flow through the bleed tube caused by variations in the pressure at which the test subject is blowing.

Such apparatus does not require use of a diverter valve to selectively direct breath toward and away from the vicinity of the sensor. Further the sensor cannot be circumvented by blocking a breath outlet.

A pressure sensor such as a pressure switch determines whether at least the predetermined minimum pressure is present within the second chamber to insure a deep lung sample is provided.

The inlet of the bleed passageway may be disposed within the wall of the second chamber at a location where the stream of breath flowing into the second chamber will not impinge directly on it. This helps to prevent changes in the amount of flow through the bleed passageway induced by changes in velocity of the breath entering the second chamber. It also helps to prevent any fluid or particulate contamination from entering the bleed passageway and possibly blocking it.

At least a part of the sensor chamber may partially block off the conduit downstream of its inlet to define parts of the first restricted passageway and the second chamber. The bleed passageway is then formed as an orifice in a wall of the sample chamber that is common to both the sample chamber and the second chamber. This arrangement is advantageous in that the sensor can be mounted in close proximity to the second chamber. The sensor therefore responds quickly to changes in the alcohol concentration occurring in the second chamber since the bleed passageway defined by the orifice is very short and has a small volume.

The pressure sensing means is a differential pressure sensing means which may be connected across the second restricted passageway between the second chamber and the exhaust vent. Should the exhaust vent become blocked off either accidentally or deliberately for the purpose of circumventing a test, the differential pressure sensing means will not indicate a flow sufficient to commence measurement of the sampling interval. By requiring the presence of an actual sufficient flow as indicated by a differential pressure, a measurement based upon a deep lung sample is more positively ensured.

To avoid possible blockage of the bleed passageway by ice formed by condensed breath in cold weather, a heater can be provided within the second chamber.

The present invention will now be described by way of example with reference to the drawings in which:

Fig. 1 shows a side cross sectional view of a preferred embodiment of a sampling head constructed according to the present invention. An interlock control module and vehicle ignition system are also represented schematically connected to the sampling head.

Fig. 2 is a view taken along line 2-2 of Fig. 1.

Fig. 3 is an electrical schematic diagram illustrating the curcuitry contained within the sampling head of Fig. 1.

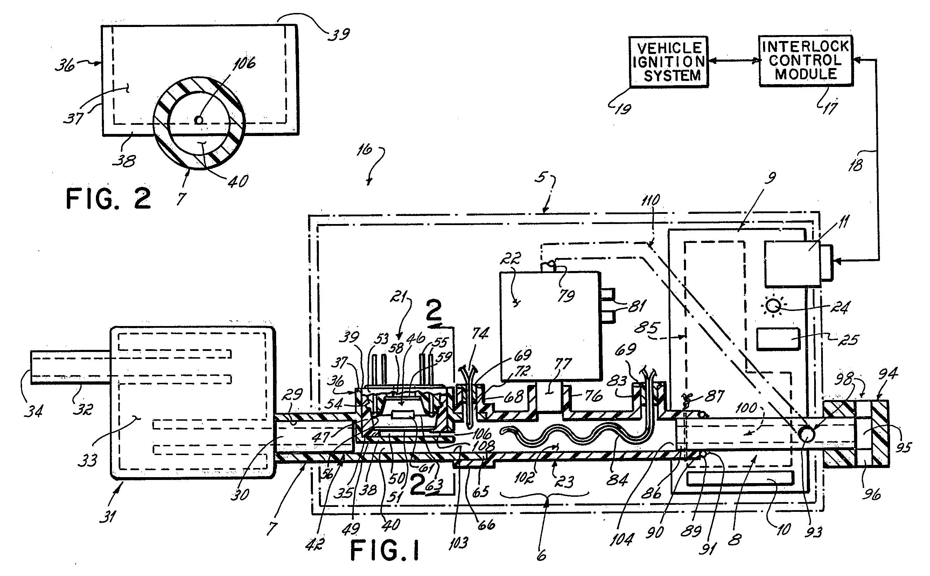

In the embodiment of Fig. 1 a sampling head 16 is remotely connected to an interlock control module 17 by way of a multiconductor cable 18. Cable 18 is preferably a coilably retractable type having a detachable modular plug connector at each end for connection between sampling head 16 and control module 17. Control module 17 together with sampling head 16 are mounted within a vehicle and connected to a vehicle's ignition system 19 to function as a vehicle interlock to prevent the vehicle from being started if a test subject/prospective driver fails a breath alcohol test.

Sampling head 16 includes a housing 5 that is spanned by a breath conduit 6 which is conveniently formed by joining inlet section 7 to the upstream end of a manifold 23 whose downstream end is joined to an exhaust tube 8. Manifold 23 communicates with a solid state gas sensor assembly 21 as well as with a pressure sensor 22. Gas sensor assembly 21 generates an electrical output signal which indicates the concentration of alcohol vapor in the breath flowing through manifold 23. Pressure sensor 22 cooperates with timing means associated with control module 17 to help insure the alcohol measurement is based on a deep lung breath sample. This is accomplished by programming module 17 to read the electrical output signal from gas sensor 21 only when pressure sensor 22 indicates that at least a predetermined minimum pressure has been maintained inside manifold 21 without interruption for at least a predetermined interval of time which shall be referred to as the sampling interval. The predetermined minimum pressure indicated by pressure sensor 22 corresponds to a flow rate of breath through manifold 23 that is sufficient to ensure a deep lung sample if that flow is maintained for the entire sampling interval.

Housing 5 further includes a printed circuit board 9 which supports a "Ready" LED 24 that is visible exteriorly of housing 5 to indicate when the system is ready to receive or is in the process of receiving a breath sample. Also mounted on circuit board 9 is a calibration potentiometer 25 for calibrating the response of sensor 21 to a particular concentration of alcohol vapor. A terminal strip 10 facilitates wiring connections (not shown) between circuit board 9 and the other electrical components within housing 5 such as gas sensor 21 and pressure sensor 22. A modular jack 11 secured to circuit board 9 and accessible exteriorly of housing 5 permits connection of circuit board 9 to control module 17 by way of cable 18.

The detailed construction and operation of control module 17 and its electrical connections to the components within sampling head 16 and a vehicle ignition 19 form no part of the present invention and are described and shown only to the extent necessary to provide a basic understanding of one type of system in which the present invention can be used. Those desiring more information regarding the above details are referred to the above-referenced U.S. patent application Serial No. 06/907,881 which has been incorporated by reference.

Inlet section 7 includes a cylindrical throat 29 which projects partially outside housing 5. Throat 29 has an inside diameter of about 0.25 (one quarter) inches and a length of about 0.75 (three quarters) inches. Throat 29 receives an outlet tube 30 of a mouthpiece 31 which can be removeably inserted into throat 29 in leak-tight engagement therewith.

Mouthpiece 31 is constructed of clear plastic and further includes an inlet tube 32 the center of which is axially offset by about one half inch from the centre of outlet tube 30 and communicates therewith through the interior of a housing 33 having interior dimensions of about 1.0 (one) inch by 1.0 (one) inch by 0.35 inches. Inlet tube 32 and outlet tube 30 are substantially identical, each having an overall length of about 1.35 inches and an inside diameter of about 0.125 (one eighth) inches. A suitable mouthpiece 31 is part number 5120 available from A.C.S. of Cincinnati, Ohio. Inlet tube 32 includes an inlet opening 34 into which a test subject blows to deliver a breath sample. Mouthpiece 31 helps to trap any spit or particulates which the subject might expel while blowing and is preferably disposable so that a number of persons can use sampling head 16 hygienically.

Referring additionally now to Fig. 2, inlet section 7 further includes a cup member 36 having a generally cylindrical side wall 37, a closed, flat bottom 38 and an open top end 39. Cup member 36 intersects tubular inlet section 7 and is formed contiguously therewith such that part of the side wall 37 of cup 36 projects interiorly of section 7 to define a restricted passage 40 of semicircular cross section. Passage 40 has a length equal to the outside diameter of cup member 36 (about 0.75 (three quarters) inches) and a cross-sectional area of about 0.015 square inches. Passage 40 defines part of a first restricted passageway 42 which extends upstream therefrom to include the interior of mouthpiece 31 to a point just downstream of its inlet opening 34.

Solid state gas sensor assembly 21 is press fitted into cup member 36 to define a sensor chamber 46. One preferred device for use as a sensor assembly 21 is a semiconductor type such as model TGS-813P made by Figaro Engineering, Inc. of Osaka, Japan and includes a generally cylindrical, hollow sensor housing 47 having a circular rim 49 that defines a circular inlet aperture 50 about 0.4 (four tenths) inches in diameter. RIM 49 is about 0.05 (five hundreths) inches high, rests on the bottom 38 of cup member 36 and supports a first screen 51 of stainless steel gauze. A base 53 snap fits into housing 47 by means of an annular detent 54. Six terminal pins 55 (only four of which are shown in Fig. 1) having contact pads 56 connected thereto are set longitudinally in base 53 at circumferentially spaced positions. Base 53 includes a circular outlet aperture 58 about 0.125inches in diameter which is covered by a second stainless steel gauze screen 59. Both screens 51 and 59 are of a double mesh type having normal dimensions of 149 micrometers and are manufactured in accordance with SUS 316-2W and JIS Z-8801. Sensing chamber 46 includes the open volume between screens 51 and 59 as well as the volume defined by the rim 49 surrounding inlet aperture 50. The open volume of sensing chamber 46 is therefore on the order of only about 0.5 millimeters.

Sensor assembly 21 further includes a sensing element 61 which, for the above-mentioned TGS-813P sensor, comprises a body of doped $SnO_2$ disposed on a small ceramic tube (not shown) the interior of which is transversed by a heater coil (also not shown). Element 61 is centrally suspended between screens 51 and 59 by six electrical leads 63 (only two shown). One end of each lead 63 is terminated upon a respective one of contact pads 56. Two of the leads 63 have their opposite ends connected to the heater coil associated with element 61 while the remaining 4 terminate on element 61 itself for sensing purposes. Each of terminal pins 55 is electrically connected to terminal strip 10 by way of wires (not shown) and are electrically connected by way of printed circuit board 9, jack 11 and cable 18 to control module 17 as described in above-incorporated U.S. Patent Application Serial No. 06/907,881 with particular reference to Fig. 2 thereof.

During alcohol sensing, control module 17 supplies the heater coil associated with sensor 21 with a current selected to maintain semiconductor element 61 within a desired temperature range of about 390°C to about 460°C and preferably closer to 420°C to 440°C for best accuracy. If element 61 is not substantially within at least the larger of the above temperature ranges, inaccurate measurements can result. Prior to a measurement, element 61 is purged to restore its electrical output signal to its equilibrium level, i.e., a level substantially corresponding to zero percent alcohol concentration. This is accomplished by control module 17 energizing the heater coil of sensor assembly 21 to a temperature which is significantly higher than 460°C for a time sufficient to cause any alcohol or other impurities adsorbed on the surface of element 61 to be oxidized and desorbed therefrom.

Inlet tube 7 terminates in a stem 65 which is press fitted into a flange 66 formed on the upstream side of manifold 23 to form an air-tight connection therewith. Flange 66 includes a first nipple 68 which receives an apertured bushing 69. Stem 65 includes a hole 72 aligned with aperture in bushing 69. A thermistor 74 is inserted through bushing 69 and hole 72 for a purpose to be explained later. For the present, it is sufficient to note that thermistor 74 serves the auxiliary purpose of preventing inlet section 7 and intermediate manifold section 23 from being pulled apart axially by operating in a fashion akin to a cotter pin.

Manifold 23 is generally cylindrical having a major internal diameter of about 0.25 (one quarter) inches and further includes a second nipple 76 which receives pressure sensor 22. Sensor 22 can be any suitable pressure sensor such as a solid state type but suitably takes the form of a differential pressure switch having a "high" pressure sensing inlet 77 disposed within second nipple 76 to sense the pressure inside manifold 23. A "low" pressure sensing inlet 79 of pressure sensor 22 communicates with the interior region of housing 5 which is at atmospheric pressure due to vents (not shown) penetrating the walls of housing 5. Pressure switch 22 also includes a pair of normally open terminals 81 which are electrically switched in accordance with the differential pressure between inlets 77 and 79. A suitable pressure sensor 22 is an electromechanical pressure switch such as model PSF-100-4.0 manufactured by World Magnetics of Traverse City, Mitchigan. This sensor causes the switching state across terminals 81 to change at a gauge pressure threshold of about 4.0 inches of water.

Intermediate manifold section 23 further includes a third nipple 83 which is also fitted with a bushing 69 which receives a heater 84. Heater 84 is constructed by inserting a length of #36 A.W.G NiCr heater wire inside a length of PTFE insulating tubing having an outside diameter of about 0.03 (three hundredths) inches. The tubing is then folded sharply back on itself in half to form a tight loop which is inserted past bushing 69 and nipple 83 to the interior of intermediate section 23. The looped end of heater 84 is grasped inside section 23 with the aid of a long-nosed pliers or similar tool inserted from the downstream end of manifold 23. The looped end of heater is then twisted while being pushed upstream toward flange 66 to form an approximately helical coil of 2 to 3 turns which are preferably disposed closely about the interior walls of intermediate manifold section 23.

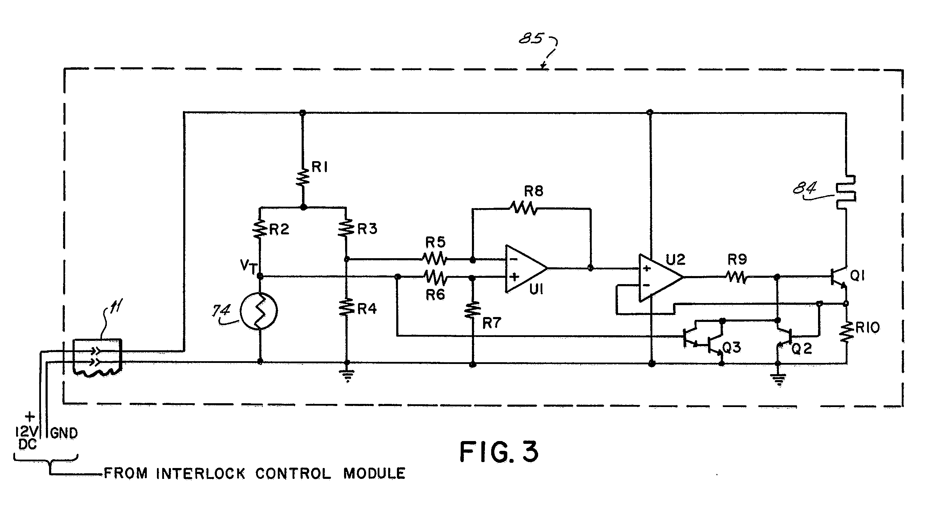

Under the control of thermistor 74 when the temperature inside manifold 23 is sufficiently cold, heater 84 is energized as necessary to warm the space within housing 5 enough to avoid an excessively cold operating environment for sensor 21 as well as to maintain a sufficient temperature to prevent freezing of the moisture in the breath passing though manifold 23. To accomplish this, thermistor 74 and heater 84 are both connected by means of wires (not shown) through terminal strip 10 to a closed-loop heater control circuit 85 mounted on circuit board 9. The structure and operation of heater control circuit 85 will now be described with reference to Fig. 3.

In the heater control circuit 85, thermistor 74 is connected in series with 150 K ohm resistor R1 and 8.2K resistor R2 across $+12$ Volt DC and ground lines emanating from control module 17 by way of a portion of jack 11 to firm a voltage divider at not $V_T$. Thermistor 74 is a negative thermal coefficient type such as part number GB41M2 made by Fenwal Electronics Div. of Kidde, Inc. of Framingham, Massachesetts. Node $V_T$ is connected, by way of a series 4.7L ohm resistor, R6 to the noninverting input of a first amplifier, U1 which itself is connected to ground by way of a 220K ohm resistor R7. The inverting input of amplifier U1 is connected by way of a series connected 4.7K resistor, R5 to a voltage divider formed by the series combination of R1 together with an 8.2K ohm resistor, R3 and an 12K ohm resistor, R4 as shown. A 220K ohm feedback resistor, R8 connects the inverting input of amp U1 with its output. Thus, U1 is configured as a conventional balanced differential amp which amplifies the voltage difference appearing between node $V_T$ and the reference voltage node located between R3 and R4. That voltage difference increases as the temperature sensed by thermistor 74 decreases. The output of amp U1 is connected to the noninverting input of a second amp U2 configured as a closed loop negative feedback amplifier whose inverting input is connected to the emitter of a transistor Q1 which is preferably an NPN type TIP 29 manufactured by Motorola Semiconductor Products, Inc. of Austin, Texas. The base of transistor Q1 is connected to the output of an amplifier U2 by way of a 3.3K ohm resistor, R9 while the collector of Q1 is connected to one end of heater 84 whose other side is connected to the $+12$ V.D.C. supply. A 1.5 ohm, 2 watt resistor, R10 connects the emitter of transistor Q1 to ground. The noninverting input of U2 is connected to the output of U1 while the inverting input of U2 is connected to the emitter of Q1. This limits the current through heater 84. A second transistor, Q2 such as an NPN type 2N3904 has its base connected to the emitter of transistor Q1, its collector to the base of Q1 and its emitter to ground. A Darlington transistor, Q3 such as a type MPSA 14 in turn has its base connected to node $V_T$, its emitter grounded and its collector connected to the collector of Q2 and the base of Q1.

In normal operation of circuit 85, the voltage at $V_T$ rises as the temperature sensed by thermistor 74 drops and is amplified by amps U1 and U2 energizing heater 84 with the amount of power required to maintain the temperature set by resistor R4.

In the event thermistor 74 should open circuit, $V_T$ is pulled up to a voltage that is sufficiently high to turn Q3 on. This prevents heater 84 from being powered. In the event heater 84 shorts out, the abnormally high current passing through Q1 would develop a sufficient voltage across R10 to bring Q2 into a conducting state, clamping the base of Q1 low and limiting the power to heater 84.

Referring again to Fig. 1 the downstream end of manifold 23 receives the upstream end of exhaust tube 8 which comprises a clear cylindrical butyrate tube about 1.375 inches long and having an inside diameter of about 0.125 inches and an outside diameter of about 0.256 inches. Tube 8 is inserted about 0.25 inches into manifold 23 and is secured thereto by means of a fine wire 87 which is passed through a series of aligned apertures in manifold 23 and tube 8 and twisted at its ends to secure tube 8 inside manifold 23. Wire 87 is preferably A.W.G. #26 and is of corrosion resistant material such as stainless steel. To assure a air-tight seal, an O-ring 89 is fitted around tube 8 in abutment with the end face 90 of manifold 23. O-Ring 89 is retained against end face 90 by means of a bead of epoxy 91 applied between the opposite side of O-Ring 89 and tube 8 as shown.

Exhaust tube 8 is provided with an internal vent 93 about 0.125 inches in diameter connecting the interior of conduit 6 with the interior of housing 5 which, as previously noted is vented to atmosphere. The portion of tube 8 extending beyond housing 5 is fitted with a plastic vent cap 94 having a longitudinal well 95 adapted to receive the end portion of exhaust tube 8 to which cap 94 is secured by solvent bonding, adhesive or other suitable means. Well 95 is transversely intersected by a cylindrical external vent bore 96 which is about 0.5 (one half) inches long and about .1875 (3/16ths) inches in diameter. Vent bore 96 communicates with the interior of tube 8 which terminates just at the edge of vent bore 96 to avoid even partially blocking it. External vent bore 96 and internal vent 93 together serve as an exhaust 98 through which most of the breath entering inlet opening 34 is finally vented to atmosphere.

Since exhaust tube 8 has an inside diameter which is smaller than that of manifold 23, the portion of exhaust tube 8 extending from its upstream end 86 to vent 93 defines a second restricted passageway 100. A second chamber 102 can be defined as the region within manifold 23 disposed between first and second restricted passageways 42 and 100 respectively. Second chamber 102 is bounded at its upstream end 103 by the downstream end of passageway 40 and is bounded at its downstream end 104 by the upstream end 86 of tube 8. Second chamber 102 has an overall length of about 2.0 (two) inches and is generally cylindrically shaped defining a total volume of about 0.1 (one tenth) cubic inches.

According to one aspect of the invention, second chamber 102 communicates with sensor chamber 46 by way of a bleed passageway 106. Bleed passageway 106 is sized to have a relatively large resistance to flow such that the bleed flow flowing from second chamber 102 to sensor chamber 46 is a small fraction of the total amount of breath blown into inlet 34 by the test subject. For instance, bleed passageway 106 preferably takes the form of a cylindrical hole about 1/64th (one sixtyfourth) of an inch in diameter disposed in that portion of the wall 37 of cup member 36 which is common to both sensor chamber 46 and second chamber 102 and lies just above the bottom 35 of cup member 36 as clearly shown in Figs. 1 and 2. So that the bleed flow from bleed passageway 106 can enter sensor chamber 46 without being obstructed by the rim 49 of sensor housing 47, rim 49 is provided with a relieved portion or notch 108 which is aligned with bleed passageway and oversized relative the diameter thereof.

The sizing and configuration of the various portions of the breath flow path as described in detail above inherently provide highly desirable flow characteristics which will now be described to afford a more complete understanding of the invention.

As pointed out earlier, a primary requirement of any breath alcohol measuring system is that it must allow sufficient breath flow so that a deep lung sample can be obtained. This is accomplished by selecting the total resistance to breath flow such that an average person blowing with moderate effort into inlet 34 can deliver at least a minimum flow of breath sufficient to yield a deep lung sample within a predetermined sampling interval. In the above embodiment for example, a person blowing into inlet 34 at a pressure of at least about twelve inches of water will induce a pressure drop of at least about three inches of water across mouthpiece 31 resulting in a minimum head of about nine inches of water at the upstream side of passageway 40. Such pressures are sufficient to allow the person to deliver a deep lung sample within a sampling interval of about 4.5 seconds. This corresponds to a minimum desired flow rate of about sixteen litres per minute entering inlet 34.

A second requirement is that the system be capable of detecting whether the above-described minimum flow is present for the entire sampling interval to ensure a deep lung sample is delivered. This is accomplished by selecting the resistance to flow entering second chamber 102 by way of first restricted passageway 42 and the resistance to the flow exiting second chamber 102 by way of bleed passageway 106 and second restricted passageway 100 so that when at least the above-referenced minimum desired flow rate is present, at least a predetermined minimum pressure exists within second chamber 102 which can be sensed by pressure switch 22. In the preferred embodiment described above, pressure switch 22 is calibrated to change state when the pressure within second chamber 102 crosses a threshold of about 4.0 inches of water. As mentioned earlier, control module 17 is equipped with timing means that begins timing the 4.5 second sampling interval when the pressure in second chamber initially rises about 4.0 inches of water. Should the pressure drop below that value before the sampling interval is completed, as would indicate a shallow exhalation or discontinuous puffing of mouth air, module 17 requires a new breath sample since a valid deep lung sample may not have been provided.

In addition to the above, the invention provides a bleed flow through bleed passageway 106 that is large enough to ensure that sensor chamber 46 is supplied with an influx of breath from manifold 23 that is sufficient relative the volume of sensor chamber 46 that the alcohol concentration within sensor chamber 46 rapidly tracks the alcohol concentration of the bleed flow thereby ensuring an accurate alcohol measurement. One can appreciate that as the bleed flow just begins its transition from upper respiratory breath, having a relatively low alcohol concentration, to deep lung breath, sensor chamber 46 will be initially filled with the breath of low alcohol concentration. If the bleed flow through passageway 106 were too small and the alcohol level of the deep lung breath were significantly higher than the gas initially filling sensor chamber 46, the alcohol reading of sensor 21 would be erroneously low for some time due to the dilution of the higher alcohol deep lung breath within sensor chamber 46. It is also important to keep the volume of sensor chamber 46 as small as practicable so that the bleed flow is not overly diluted therein. To avoid this "starving" of sensor 21 in the preferred embodiment described above, the 1/64 inch diameter bleed passageway 106 is large enough to provide a sufficient minimum bleed flow (i.e., about three hundred millilitres per minute) to sensor chamber 46. This flow is sufficient relative the volume of sensor chamber 46 so that when the pressure in second chamber 102 is maintained at at least the predetermined minimum pressure of about 4.0 inches of water, the output of sensor 21 is accurately correlated to the true alcohol content of the deep lung breath by the end of the sampling interval of about 4.5 seconds.

While the bleed flow through bleed passageway 106 must at least equal the minimum bleed flow described above, it is of equal importance that the bleed flow not be large enough to cool the sensing element 61 of sensor 21 below the range of temperatures within which sensor 21 operates with accuracy. This is accomplished by selecting the flow resistance through first and second restricted passageways 42 and 100 in conjunction with the size of bleed passageway 106 such that when a normal person blows with a maximum expected pressure (about 21 inches of water) into inlet 34, the pressure within second chamber 102 does not exceed a predetermined maximum pressure which would be sufficient to induce a bleed flow large enough to cool element 61 below its accurate operating temperature range. For example, in the preferred embodiment described in detail above, it is extremely difficult for a person to blow into inlet 34 hard enough to raise the pressure inside second chamber beyond a maximum expected value of about 6 (six) inches of water. Given the size of bleed passageway 106, such a pressure is not high enough to produce a bleed flow sufficient to cool element 61.

In light of the above, it can be appreciated that the size and configuration of the breath flow path through the system operate to effectively regulate the pressure within second chamber 102 and therefore the bleed flow through bleed passageway 106. While the gauge pressure at inlet 34 can vary between zero inches of water (when no one is blowing therein) up to about 21 inches of water (when someone is blowing very forcefully) the pressure inside second chamber 102 is limited to a maximum of about 6 (six) inches of water due to the pressure drop across first restricted passageway 42 and the escape of breath through tube 8. On the other hand, the low end of the pressure range within second chamber 102 is effectively limited to about 4 inches of water as required by pressure sensor 22 to ensure a valid deep lung sample is obtained. Therefore, during sampling the pressure inside second chamber 102 is held constant to within about 2 (two) inches of water. This in turn helps to ensure that the bleed flow to sensor chamber 46 remains fairly constant. By so reducing fluctuations in the bleed flow through bleed passageway 106, erratic changes in both the temperature of sensor element 61 and the alcohol concentration in its vicinity are avoided thereby improving the accuracy of measurement. Fluctuations in the bleed flow are further reduced due to the location of bleed passageway 106 relative to second chamber 102.

As can be seen from Fig. 1, the high velocity breath stream entering second chamber 102 will have a principal velocity component directed downstream parallel to the longitudinal axis of passageway 40. Due to the position of bleed passageway 106 in the wall 37 of cup 36 just above the downstream end of passageway 40, the above velocity component is directed 180 degrees away from the direction of the bleed flow entering bleed passageway 106 and flowing toward the left in Fig. 1. Therefore, that part of the breath entering bleed passageway 106 from second chamber 102 must eventually reverse direction and enter bleed passageway 106 in a back-bleed fashion. For this reason, the bleed flow entering sensor chamber 46 through bleed passageway 106 will depend primarily on the static pressure within second chamber 102 and will not be influenced significantly by instantaneous changes in the velocity of breath entering second chamber 102 from passageway 40 except to the extent such velocity variations are ultimately reflected in changes in the static pressure in second chamber 102.

Another advantage of the above-described apparatus is that sensor chamber 46 is located in close proximity to second chamber 102 and the bleed passageway 106 joining the two chambers 46 and 102 has a small volume. Accordingly, any changes in the alcohol concentration of breath in the second chamber 102 are rapidly reflected in corresponding changes in the alcohol concentration within sensor chamber 46. This permits electrical output signal generated by sensor element 61 to more accurately track the alcohol concentration of the breath in second chamber 102.

In operation, once sensor element 61 has been purged and is at a temperature within its desired operating range of 390°C to 460°C and preferably within 420°C to 440°C, control module 17 turns on "Ready" LED 24 to indicate to the test subject to begin blowing into the inlet 34 of mouthpiece 31. When blowing commences, breath flows from inlet 34 toward second chamber 102. As it does so, a substantial pressure drop occurs across first restricted passageway 40. This limits the pressure that can build up inside second chamber 102 to a maximum expected value. Assuming the test subject is blowing at a sufficient rate, second chamber 102 fills with breath, most of which continues to flow downstream through the second restricted flow passage 100 of exhaust tube 8. Due to the restriction at second passage 100, the pressure within second chamber 102 increases until it reaches at least a predetermined minimum pressure, corresponding to a minimum flow sufficient to provide a deep lung sample if maintained for a predetermined sampling interval such as 4.5 seconds. When that predetermined minimum pressure is reached, pressure sensor 22 so indicates by changing the switching state across contacts 81. Control module 17 responds by initiating timing of the sampling interval and turning off "Ready" LED 24 thereby indicating to the test subject that sufficient breath flow is taking place.

The majority of the breath entering second chamber 102 will flow into exhaust tube 8 to be expelled from exhaust 98 partially outside housing 5 by way of vent bore 96 and partially inside housing 5 by way of vent 93. However, a small fraction of the breath entering second chamber 102 will bleed backward into bleed passageway 106 to create a bleed flow therein. The precise fraction of the total breath flow which goes to make up the bleed flow has been measured to be about 2% of the total volume of breath entering inlet 34. The bleed flow exits bleed passageway 106 and enters sensor chamber 46 with its principal velocity component directed to the left in Fig. 1 generally parallel to the axis of bleed passageway 106. The breath then flows upward through first screen 51 which helps to diffuse the flow in the area of sensor element 61. The breath flows past element 61 and exits sensor chamber 46 by way of second screen 59. The flow through chamber 46 is promoted by the pressure gradient across sensor chamber 46 due to the influx of the bleed flow as well as by the thermal gradient induced by the heater associated with sensor element 61.

The surface of sensor element 61 absorbs any alcohol which may be present according to its concentration in the vicinity of sensor element 61. This causes the conductivity of element 61 to change thereby generating an electrical signal which is transmitted to control module 17 by way of a pair of terminal pins 55, connected by wires (not shown) to terminal strip 10, printed circuit board 9, jack 11 and cable 18. When the timing means associated with control module 17 indicates that the sample interval is at an end, provided the pressure sensed by pressure sensor 22 has remained above its predetermined minimum value for the entire sampling interval, control module 17 reads the electrical signal emanating from sensor element 61 and determines the alcohol concentration it represents in a manner well known in the art. In the event the alcohol concentration exceeds a predetermined limit, control module 17 disables vehicle ignition system 19 to prevent starting of the vehicle.

In the event thermistor 74 senses a suitably cold temperature, heater 84 is operated by circuit 85 in the manner described earlier in order to prevent freezing of moisture within breath conduit 6 and particularly within second chamber 102. This avoids any possibility of bleed passageway 106 icing partially or completely closed and causing erroneous alcohol indications. Due to the close proximity of sensor element 61 to bleed passageway 106, the heater (not shown) associated with element 61 is normally sufficient to prevent such icing except at temperatures well below 0°F at which time it is assisted by heater 84.

It is noted that in the event external vent bore 96 were to be blocked in an attempt to evade testing or by accident, it would not be possible to blow into inlet 34 and maintain the predetermined minimum pressure sensed by pressure switch 22 without sufficient flow through inlet 34 to yield a deep lung sample. This is so because internal exhaust vent 94 would still permit breath to be freely vented inside housing 5 which itself is vented to atmosphere. While this technique is effective, it may be considered undesirable to vent large amounts of moist air within the confines of housing 5. This is a particular concern in colder weather when a buildup of excessive amounts of condensation could possibly interfere with certain electrical components such as those mounted on circuit board 9.

One way to deal with the above concern is illustrated in Fig. 1. As shown in dashed lines, a length of plastic tubing 110 can be connected between the low pressure sensing inlet 79 of pressure switch 22 and internal vent 93. This causes pressure switch 22 to respond not to the pressure between second chamber 102 and atmosphere but rather to the differential pressure drop across second restricted flow passage 100. In that case, if vent bore 96 were blocked, no flow would take place through passage 100 and no pressure drop would appear thereacross. This would prevent pressure sensor 22 from sensing a pressure that does not truly indicate a sufficient flow to ensure a deep lung sample. In the event that the differential pressure across passage 100 differed slightly from the gauge pressure inside second chamber 102 when the predetermined minimum flow sufficient to ensure a deep lung sample was being delivered into inlet 34, the calibration of sensor 22 could be adjusted accordingly. This would permit contacts 81 to change state at the proper predetermined minimum level of flow.

While the invention has been illustrated and described for use in a vehicle interlock system this invention is applicable to any gas analysis system wherein a gas sample, such as a breath sample is delivered to a sensor for determination of the presence or concentration of a constituent such as alcohol.

## Claims

1. Apparatus for determining the presence or concentration of a constituent, such as alcohol, in a gas, such as breath, sample by delivering the sample to a sensor characterised in that a chamber (102) is formed in a conduit (6) for breath flow there being a first restricted passage (40) located between the chamber (102) and an inlet (7), a second restricted passage (100) located between the chamber (102) and an exhaust vent (8), the chamber (102) being connected via a bleed passage (106) to a sensor chamber (46) which substantially envelops a sensor (61), the bleed passage (106) conducting a fraction of the flow of breath entering the inlet (7) to the sensor chamber (46) and in that differential pressure sensing means (22) is connected to the chamber (102) to sense the pressure in the chamber (102), the arrangement being such that if and only if the pressure remains within a predetermined pressure range during a predetermined sampling interval, a signal relating to the concentration of the sample in contact with the sensor (61), is emitted at the end of the sampling interval.

2. Apparatus as claimed in claim 1 wherein the first and second flow passages (40, 100) are designed to create at least a predetermined minimum pressure within the chamber (102) when the flow of breath through the inlet (7) at least equals a minimum flow, said minimum flow being selected to ensure that a deep lung breath sample will be provided when the minimum flow is continued without interruption for a period at least equal to a interruption for a period at least equal to a predetermined sampling interval, the size of the first restricted flow passage (40) being further selected to ensure that the pressure within the second chamber (102) does not exceed a predetermined maximum pressure, provided the pressure at the inlet (7) does not exceed a maximum expected value.

3. Apparatus as claimed in claim 2 wherein the bleed passage (106) is sized to provide a bleed flow from the second chamber (102) to the sample chamber (46) which, when the pressure within said second chamber does not fall below said minimum pressure, is sufficient to insure that the electrical output signal generated by the sensor (61) at the end of the sampling interval reaches a level which accurately reflects the concentration of alcohol in the deep lung breath sample, the level being substantially independent of instantaneous variations in the bleed flow occurring over the sample interval, and, when the pressure within the second chamber (102) does not exceed the predetermined maximum pressure, is insufficient to cool the sensor (61) enough that the electrical output signal is no longer substantially correlated with the true concentration of alcohol in the deep ling breath sample.

4. Apparatus as claimed in any of claims 1-3 wherein said sensor chamber comprises a cup member (36) at least a portion of which intersects the conduit (6) to define at least a portion of the first restricted flow passage (40) and the second chamber (102) and wherein the bleed passageway (106) is located in a wall common to both the second chamber (102) and the cup (36) whereby mounting of the sensor (61) in close proximity of the second chamber (102) is achieved.

5. Apparatus as claimed in any preceeding claim wherein the pressure sensing means (22) comprises a pressure switch which changes state when the pressure in the chamber (102) reaches a predetermined threshold value.

6. Apparatus as claimed in any preceeding claim including a heater (84) thermally connected with the second chamber (102) for preventing the freezing of breath condensed therein.

7. Apparatus as claimed in any preceeding claim further comprising temperature sensing means (74) disposed in temperature sensing relation with the interior of the second chamber (102).

8. Apparatus as claimed in any preceeding claim further comprising differential pressure sensing means (22) disposed between the chamber (102) and either the upstream side of said first restricted passageway (40) or the downstream side of said second restricted passageway (100).

INTERLOCK CONTROL MODULE

VEHICLE IGNITION SYSTEM

FIG. 1

FIG. 2

FIG. 3

FROM INTERLOCK CONTROL MODULE

Q1, R10, Q2, Q3, R9, U2, 84, 85, R8, U1, R5, R6, R7, R3, R4, R1, R2, $V_T$, 74, 11, +12V DC, GND